# EUROPÄISCHE PATENTSCHRIFT

(11) **EP 1 817 108 B1**
(45) Veröffentlichungstag und Bekanntmachung des Hinweises auf die Patenterteilung: **04.06.2008**
(21) Anmeldenummer: 05798767.9
(22) Anmeldetag: 12.10.2005
(51) Int. Cl.: B01J 31/40, B01J 38/56, B01D 11/04, C07C 253/10, C07C 253/32, B01J 31/18, B01J 31/24

(54) **EXTRAKTION VON NICKEL(0)-KOMPLEXEN AUS NITRILGEMISCHEN MIT VERMINDERTER MULMBILDUNG**
EXTRACTION OF NICKEL(0) COMPLEXES FROM NITRILE MIXTURES WITH REDUCED POWDER FORMATION
EXTRACTION DE COMPLEXES DE NICKEL(0) CONTENUS DANS DES MELANGES DE NITRILES AVEC FORMATION REDUITE DE MATIERE PULVERULENTE

(30) Priorität: 18.10.2004 DE 102004050935
(43) Veröffentlichungstag der Anmeldung: 15.08.2007
(73) Patentinhaber: BASF SE, 67056 Ludwigshafen (DE)
(72) Erfinder: DECKERT, Petra, 69245 Bammental (DE); BASSLER, Peter, 68519 Viernheim (DE); BARTSCH, Michael, 67433 Neustadt (DE); HADERLEIN, Gerd, 67269 Grünstadt (DE); LUYKEN, Hermann, 67069 Ludwigshafen (DE); SCHEIDEL, Jens, 69493 Hirschberg (DE); PFAB, Peter, 67433 Neustadt (DE); AECHTNER, Tobias, 68163 Mannheim (DE); SIEGEL, Wolfgang, 67117 Limburgerhof (DE)
(86) Internationale Anmeldenummer: PCT/EP2005/010956
(87) Internationale Veröffentlichungsnummer: WO 2006/042675

(56) Entgegenhaltungen:
- EP-A- 0 464 691
- WO-A-20/05073174
- DE-A1- 2 154 501
- DE-A1- 3 235 433
- DE-A1- 3 247 292
- DE-A1- 10 311 122
- DE-A1-3102004 004 68
- US-A- 3 773 809
- US-A- 4 339 395
- US-A1- 2004 140 263
- BOHNET M ET AL (HRSG.): "Ullmann's Encyclopedia of Industrial Chemistry" [Online] 2002, WILEY-VCH , WEINHEIM, DE , XP002379061 Gefunden im Internet: URL:http://www.mrw.interscience.wiley.com/ ueic/articles/b03_06/pdf_fs.html> [gefunden am 2005-04-27] Elektronische Version: Müller, Eckart; et al. "Liquid?Liquid Extraction", Seiten 1-54; Posting Date 15-06-2000 Seiten 10-11 Seite 37 Seiten 39-41
- GILL, DIP SINGH ET AL: "Copper quadrupole coupling constants in binary mixtures of acetonitrile with some other nitriles at 298 K" JOURNAL OF MOLECULAR LIQUIDS , 111(1-3), 85-93 CODEN: JMLIDT; ISSN: 0167-7322, 5. März 2004 (2004-03-05), XP002378986

## Beschreibung

Die Erfindung betrifft ein Verfahren zur extraktiven Abtrennung von homogen gelösten Katalysatoren aus einem Reaktionsaustrag einer Hydrocyanierung von ungesättigten Mononitrilen zu Dinitrilen, durch Extraktion mittels eines Kohlenwasserstoffs K gemäß Anspruch 1.

Für Hydrocyanierungen von ungesättigten Mononitrilen sind beispielsweise Nickelkomplexe von Phosphorliganden geeignete Katalysatoren. So wird beispielsweise Adipodinitril, ein wichtiges Intermediat in der Polyamidproduktion, durch zweifache Hydrocyanierung von 1,3-Butadien hergestellt. Dabei wird in einer ersten Hydrocyanierung 1,3-Butadien mit Cyanwasserstoff in Gegenwart von Nickel(0), das mit Phosphorliganden stabilisiert ist, zu 3-Pentennitril umgesetzt. In einer zweiten Hydrocyanierung wird anschließend 3-Pentennitril mit Cyanwasserstoff zu Adipodinitril ebenfalls an einem Nickel(0)-Katalysator, allerdings gegebenenfalls unter Zusatz eines Promotors, z.B. einer Lewis-Säure, umgesetzt. Nickel(0) oder Ni(0) bedeutet Nickel in der Oxidationsstufe null.

Um die Wirtschaftlichkeit der Hydrocyanierung zu erhöhen, wird üblicherweise der Nickelkatalysator abgetrennt und zurückgeführt (Katalysatorkreislauf). Da das Katalysatorsystem in der zweiten Hydrocyanierung, das eine Mischung aus Komplex und freiem Liganden darstellt, thermisch wenig belastbar ist, kann die Abtrennung des hochsiedenden Adipodinitrils vom Katalysatorsystem nicht destillativ erfolgen. Daher wird die Trennung im Allgemeinen extraktiv mit Cyclohexan oder Methylcyclohexan oder anderen Kohlenwasserstoffen als Extraktionsmittel durchgeführt. Das Katalysatorsystem verbleibt dabei - idealerweise vollständig, unter realen Bedingungen zumindest teilweise - in der leichteren Kohlenwasserstoffphase, während die schwerere Phase polarer ist und rohes Adipodinitril und ggf. die Lewis-Säure enthält. Das Extraktionsmittel wird nach der Phasentrennung in der Regel destillativ unter vermindertem Druck abgetrennt. Der Siededruck des Extraktionsmittels ist dabei deutlich höher als der des Adipodinitrils.

Bei der Extraktion kann eine unerwünschte Mulmbildung auftreten. Unter Mulm wird ein Bereich unvollständiger Phasentrennung zwischen Ober- und Unterphase verstanden, meist ein flüssig/flüssig-Gemisch, in dem auch Feststoffe dispergiert sein können. Übermäßige Mulmbildung ist unerwünscht, da sie die Extraktion behindert und u.U. die Extraktionsvorrichtung vom Mulm geflutet werden kann, wodurch sie ihre Trennaufgabe nicht mehr erfüllen kann.

In den US-PS 3,773,809 und 5,932,772 wird die Extraktion des Katalysatorkomplexes und der Liganden mit Paraffinen und Cycloparaffinen, beispielsweise Cyclohexan, Heptan und Octan, oder Alkylaromaten, beschrieben.

Aus der US-PS 4,339,395 ist ein Verfahren zur extraktiven Aufarbeitung von Reaktionsausträgen von Hydrocyanierungen für Katalysatorsysteme mit monodentalen Liganden und einem Triarylboran als Promotor bekannt, bei dem eine geringe Menge Ammoniak zudosiert wird, um Mulmbildung zu vermeiden.

Die WO 2004/062765 beschreibt die extraktive Abtrennung eines Nickel-Diphosphit-Katalysators aus einem Gemisch von Mono- und Dinitrilen mit Alkanen oder Cycloalkanen als Extraktionsmittel, wobei das Gemisch mit einer Lewis-Base, z.B. Organoaminen oder Ammoniak, behandelt wird.

Aus der US-PS 5,847,191 ist ein Verfahren zur extraktiven Aufarbeitung von Reaktionsausträgen von Hydrocyanierungen bekannt, wobei die Chelatliganden C₉- bis C₄₀-Alkylreste tragen.

Die US-PS 4,990,645 beschreibt, dass die Extraktionsfähigkeit des Nickelkomplexes und des freien Liganden verbessert werden kann, wenn der in der Reaktion gebildete Feststoff Ni(CN)₂ vor der Extraktion in einem Dekanter abgetrennt wird. Dazu wird zuvor ein Teil des Pentennitrils abgedampft, um die Löslichkeit des Katalysators und des Ni(CN)₂ zu verringern.

Die ältere, nicht vorveröffentlichte deutsche Patentanmeldung DE 102004 045036.6 vom 15.09.2004 beschreibt die Extraktion von Ni(0)-Komplexen mit phosphorhaltigen Liganden und/oder freien phosphorhaltigen Liganden aus dem Austrag einer Hydrocyanierungsreaktion mittels eines Kohlenwasserstoffs unter bestimmten Randbedingungen. Eine Behandlung des Austrags mit unpolaren aprotischen Flüssigkeiten vor der Extraktion wird nicht erwähnt.

Der vorliegenden Erfindung lag die Aufgabe zugrunde, den geschilderten Nachteilen abzuhelfen. Es sollte ein Verfahren zur extraktiven Abtrennung von homogen gelösten Katalysatoren, z.B. Nickel(0)-Komplexen mit phosphorhaltigen Liganden und/oder freien phosphorhaltigen Liganden, aus einem Reaktionsaustrag einer Hydrocyanierung von ungesättigten Mononitrilen zu Dinitrilen bereitgestellt werden, das die zuvor beschriebenen Nachteile der bekannten Verfahren vermeidet. Insbesondere soll das Verfahren die Bildung von Mulm deutlich vermindern oder vollständig verhindern und dadurch einen ungestörten Betrieb der Extraktionsvorrichtung ermöglichen.

Dabei sollte die Wirksamkeit der Extraktion, insbesondere die Wiederfindung des Katalysators in der Kohlenwasserstoffphase, nicht vermindert werden.

Demgemäß wurde das eingangs genannte Verfahren gefunden. Bevorzugte Ausführungsformen der Erfindung sind den Unteransprüchen zu entnehmen.

Der Katalysator ist im Reaktionsaustrag homogen gelöst. Prinzipiell kommen als Katalysator alle Verbindungen in Betracht, welche die Hydrocyanierung von Mononitrilen zu Dintrilen katalysieren. Bevorzugt es handelt sich beim Katalysator um Nickel(0)-Komplexe mit phosphorhaltigen Liganden, und/oder freie phosphorhaltige Liganden. Diese Ni(0)-Komplexe und Liganden sowie ggf. verwendete Lewis-Säuren bzw. Promotoren werden weiter unten beschrieben; zunächst wird das erfindungsgemäße Verfahren erläutert.

In einer besonders bevorzugten Ausführungsform wird das erfindungsgemäße Verfahren bei der Herstellung von Adipodinitril verwendet. Somit ist das erfindungsgemäße Verfahren vorzugsweise für 3-Pentennitril als Mononitril und Adipodinitril als Dinitril bestimmt. Ebenso bevorzugt wird der Reaktionsaustrag der Hydrocyanierung durch Umsetzung von 3-Pentennitril mit Cyanwasserstoff in Gegenwart mindestens eines Nickel(0)-Komplexes mit phosphorhaltigen Liganden, gegebenenfalls in Gegenwart eines Promotors, z.B. mindestens einer Lewis-Säure, erhalten.

Das erfindungsgemäße Verfahren eignet sich zur extraktiven Abtrennung von homogen gelösten Katalysatoren, insbesondere Ni(0)-Komplexen, die phosphorhaltige Liganden und/oder freie phosphorhaltige Liganden enthalten, aus einem Reaktionsaustrag, der bei einer Hydrocyanierung von ungesättigten Mononitrilen zu Dinitrilen anfällt.

Anspruchs gemäß wird der Reaktionsaustrag in Schritt a) vor Schritt b) durch Destillation z.B. bei Drücken von 0,1 bis 5000, bevorzugt 0,5 bis 1000 und insbesondere 1 bis 200 mbar (abs.) und Temperaturen von 10 bis 150, bevorzugt 40 bis 100°C eingeengt, beispielsweise auf 5 bis 70, bevorzugt 15 bis 60 % seines ursprünglichen Volumens.

### Schritt b): Zufügen des Kohlenwasserstoffs K

In Schritt b) fügt man dem Reaktionsaustrag einen Kohlenwasserstoff K hinzu, wodurch man einen Strom I erhält. Die Verbindung K liegt zumindest unter den in Schritt b) herrschenden Druck- und Temperaturbedingungen in flüssiger Form vor K; bei anderen Druck- und Temperaturbedingungen kann K auch fest oder gasförmig sein.

### Ausgestaltung des Zufügens der Flüssigkeit

Der Kohlenwasserstoff K kann dem eingeengten Reaktionsaustrag in üblichen Mischvorrichtungen zugefügt werden. Bevorzugt weil verfahrenstechnisch besonders einfach vermischt man in Schritt b) den Kohlenwasserstoff K mit dem eingeengten Reaktionsaustrag in einem Rührbehälter oder einem Umpumpkreislauf.

Bevorzugt wird der Kohlenwasserstoff K mit dem eingeengten Reaktionsaustrag innig vermischt. Als Rührbehälter eignen sich übliche Flüssigkeitsmischer, die mit intensivmischenden Mischelementen und/oder statischen bzw. beweglichen Einbauten versehen sein können.

Die Verwendung eines Umpumpkreislaufs ist ebenfalls bevorzugt. Er wird üblicherweise derart betrieben, dass das Verhältnis von Umpumpmenge zu Ausstoß aus dem Umpumpkreis, 0,1 : 1 bis 1000 : 1, bevorzugt 1 : 1 bis 100 : 1 und besonders bevorzugt 2 : 1 bis 25 : 1 beträgt. Als Umlaufpumpe eigen sich z.B. Zahnradpumpen oder andere übliche Pumpen. Bevorzugt arbeitet die Umlaufpumpe gegen einen Überströmer, der bei einem definierten Druck von z.B. 3 bis 10 bar (abs.) öffnet.

In einer ganz besonders bevorzugten Ausführungsform ist der Kohlenwasserstoff K ein Teilstrom des Stroms 11 (mit Katalysator angereicherter Kohlenwasserstoff K, siehe unten), der bei Schritt c) anfällt. Dies bedeutet, dass in Schritt c) ein Teil von Strom II abgezweigt wird und man den abgezweigten Teil in Schritt b) dem Reaktionsaustrag zufügt. In dieser Ausführungsform wird demnach ein Teil des Stroms II im Kreis gefahren.

In einer anderen, ebenfalls bevorzugten Ausführungsform wird der Kohlenwasserstoff K direkt in eine Verweilzeitstrecke (siehe weiter unten) dosiert, beispielsweise an deren Anfang.

Das Zufügen des Kohlenwasserstoffs K erfolgt in der Regel bei Temperaturen von 0 bis 150, bevorzugt 10 bis 100 und insbesondere 20 bis 80°C, und Drücken von 0,01 bis 100, bevorzugt 0,1 bis 10 und insbesondere 0,5 bis 5 bar (abs.).

Die erforderliche Menge des Kohlenwasserstoffs K kann in weiten Grenzen varriieren. Sie ist in der Regel geringer als die verwendete Menge des Kohlenwasserstoffs K, mit dem in Schritt c) extrahiert wird, kann jedoch auch größer sein. Bevorzugt beträgt die Menge des Kohlenwasserstoffs K 0,1 bis 200 Vol.-%, insbesondere 1 bis 50 Vol.-% und besonders bevorzugt 5 bis 30 Vol.-%, bezogen auf die Menge des in Schritt c) zur Extraktion eingesetzten Kohlenwasserstoffs K.

Der zweiphasige Strom I wird ohne Separation der Phasen - d.h. beide Phasen örtlich und zeitlich zusammen - in den Extraktionsapparat geführt. Bevorzugt erfolgt dies im Gegenstrom zum Kohlenwasserstoff K (Extraktionsmittel).

### Optionale Behandlung mit Ammoniak oder Amin

In einer bevorzugten Ausführungsform des erfindungsgemäßen Verfahrens behandelt man den eingeengten Reaktionsaustrag der Hydrocyanierung vor Schritt b), bzw. den Strom I während oder nach Schritt b) oder während Schritt c), mit Ammoniak oder einem primären, sekundären oder tertiären aromatischen oder aliphatischen Amin. Aromatisch schließt alkylaromatisch, und aliphatisch schließt cycloaliphatisch ein.

Es wurde gefunden, dass sich durch diese Ammoniak- bzw. Aminbehandlung der Gehalt an-Katalysator, insbesondere an Nickel(0)-Komplex bzw. Ligand bei der Extraktion (Schritt c)) in der zweiten, mit Dinitrilen angereicherten Phase (Strom III) vermindern lässt, d.h. bei der Extraktion wird die Verteilung des Ni(0)-Komptexes bzw. der Liganden auf die beiden Phasen zugunsten der ersten Phase (Strom II) verschoben. Die Ammoniak- bzw. Aminbehandlung verbessert die Katalysatoranreicherung im Strom II; dies bedeutet geringere Katalysatorverluste im Katalysatorkreislauf und verbessert die Wirtschaftlichkeit der Hydrocyanierung.

Demnach geht in dieser Ausführungsform der Extraktion eine Behandlung des Reaktionsaustrags bzw. von Strom I mit Ammoniak bzw. einem Amin voraus oder erfolgt während der Extraktion. Dabei ist die Behandlung während der Extraktion weniger bevorzugt.

Besonders bevorzugt fügt man den Ammoniak bzw. das Amin zusammen mit dem Kohlenwasserstoff K zu. Insbesondere erfolgt die Zugabe Zugabe des Kohlenwasserstoffs K und des Ammoniaks bzw. Amins in derselben Mischvorrichtung.

Als Amine verwendet man Monomamine, Diamine, Triamine oder höherfuktionelle Amine (Polyamine). Die Monoamine weisen üblicherweise Alkylreste, Arylreste oder Arylalkylreste mit 1 bis 30 C-Atomen auf; geeignete Monoamine sind z.B. primäre Amine, z.B. Monoalkylamine, sekundäre oder tertiäre Amine, z.B. Dialkylamine. Geeignete primäre Monoamine sind beispielsweise Butylamin, Cyclohexylamin, 2-Methylcyclohexylamin, 3-Methylcyclohexylamin, 4-Methylcyclohexylamin, Benzylamin, Tetrahydrofurfurylamin und Furfurylamin. Als sekundäre Monoamine kommen z.B. Diethylamin, Dibutylamin, Di-n-propylamin und N-Methylbenzylamin in Betracht. Als tertiäre Amine eignen sich beispielsweise Trialkylamine mit C₁₋₁₀-Alkylresten, wie Trimethylamin, Triethylamin oder Tributylamin.

Als Diamine eignen sich z.B. solche der Formel R¹-NH-R²-NH-R³, worin R¹, R² und R³ unabhängig voneinander Wasserstoff oder einen Alkylrest, Arylrest oder Arylalkylrest mit 1 bis 20 C-Atomen bedeuten. Der Alkylrest kann linear oder insbesondere für R² auch cyclisch sein. Geeignete Diamine sind beispielsweise Ethylendiamin, die Propylendiamine (1,2-Diaminopropan and 1,3-Diaminopropan), N-Methyl-ethylendiamin, Piperazin, Tetramethylendiamin (1,4-Diaminobutan), N,N'-Dimethylethylendiamin, N-Ethylethylendiamin, 1,5-Diaminopentan, 1,3-Diamino-2,2-diethylpropan, 1,3-Bis-(methylamino)propan, Hexamethylendiamin (1,6-Diaminohexan), 1,5-Diamino-2-methylpentan, 3-(Propylamino)-propylamin, N,N'-Bis-(3-aminopropyl)-piperazin, N,N'-Bis-(3-aminopropyl)-piperazin und Isophorondiamin (IPDA).

Als Triamine, Tetramine bzw. höherfunktionelle Amine eignen sich z.B. Tris(2-aminoethyl)amin, Tris(2-aminopropyl)amin, Diethylentriamin (DETA), Triethylentetramin (TE-TA), Tetraethylenpentamin (TEPA), Isopropylentriamin, Dipropylentriamin und N,N'-bis(3-aminopropyl-ethylendiamin). Aminobenzylamine und Aminohydrazide mit 2 oder mehr Aminogruppen sind ebenfalls geeignet.

Naturgemäß kann man auch Mischungen von Ammoniak mit einem oder mehreren Aminen, oder Mischungen mehrerer Amine verwenden.

Bevorzugt verwendet man Ammoniak oder aliphatische Amine, insbesondere Trialkylamine mit 1 bis 10 C-Atomen im Alkylrest, z.B. Trimethylamin, Triethylamin oder Tributylamin, sowie Diamine wie Ethylendiamin, Hexamehtylendiamin oder 1,5-Diamino-2-methylpentan.

Besonders bevorzugt ist Ammoniak allein, d.h. besonders bevorzugt verwendet man neben Ammoniak kein Amin. Wasserfreier Ammoniak ist ganz besonders bevorzugt; dabei bedeutet wasserfrei einen Wassergehalt unter 1 Gew.-%, bevorzugt unter 1000 und insbesondere unter 100 ppm by weight.

Das Molverhältnis von Amin zu Ammoniak kann in weiten Grenzen variiert werden, liegt in der Regel bei 10000 : 1 bis 1 : 10000.

Die Menge des eingesetzten Ammoniaks bzw. Amins richtet sich u.a. nach Art und Menge des Katalysators, z.B. des Nickel(0)-Katalysators und/oder der Liganden, und - sofern mitverwendet - nach Art und Menge der Lewis-Säure, die bei der Hydrocyanierung als Promotor eingesetzt wird. Üblicherweise beträgt das Molverhältnis von Ammoniak bzw. Amin zu Lewis-Säure mindestens 1 : 1. Die Obergrenze dieses Molverhältnisses ist in der Regel unkritisch und beträgt beispielsweise 100 : 1; der Über schuss an Ammoniak bzw. Amin sollte jedoch nicht so groß sein, dass sich der Ni(0)-Komplex bzw. dessen Liganden zersetzen. Bevorzugt beträgt das Molverhältnis Ammoniak bzw. Amin zu Lewis-Säure 1 : 1 bis 10 : 1, besonders bevorzugt 1,5 : 1 bis 5 : 1, und insbesondere etwa 2,0 : 1. Sofern man eine Mischung aus Ammoniak und Amin verwendet, gelten diese Molverhältnisse für die Summe aus Ammoniak und Amin.

Die Temperatur bei der Behandlung mit Ammoniak bzw. Amin ist üblicherweise nicht kritisch und beträgt beispielsweise 10 bis 140, bevorzugt 20 bis 100 und insbesondere 20 bis 90°C. Auch der Druck ist in der Regel nicht kritisch.

Der Ammoniak bzw. das Amin kann dem Reaktionsaustrag gasförmig, flüssig (unter Druck stehend) oder gelöst in einem Lösungsmittel zugegeben werden. Als Lösungsmittel eignen sich z.B. Nitrile, insbesondere solche, die bei der Hydrocyanierung voniegen, und weiterhin aliphatische, cycloaliphatische oder aromatische Kohlenwasserstof fe, wie man sie bei dem erfindungsgemäßen Verfahren als Extraktionsmittel verwendet, beispielsweise Cyclohexan, Methylcyclohexan, n-Heptan oder n-Octan.

Die Ammoniak- bzw. Aminzugabe erfolgt in üblichen Vorrichtungen, beispielsweise solchen zur Gaseinleitung oder in Flüssigkeitsmischern. Der dabei in vielen Fällen ausfallende Feststoff kann entweder im Reaktionsaustrag verbleiben, d.h. der Extraktion wird eine Suspension zugeführt, oder abgetrennt werden wie nachfolgend beschrieben.

### Optionale Abtrennung der Feststoffe

In einer bevorzugten Ausführungsform werden Feststoffe, die in Schritt b) des Verfahrens ausfallen, vor der Extraktion (Schritt c)) aus dem Strom 1 abgetrennt.

Dadurch lässt sich in vielen Fällen die Extraktionsleistung des erfindungsgemäßen Verfahrens weiter verbessern, denn oftmals vermindern anfallende Feststoffe die Trennleistung der Extraktionsvorrichtungen. Außerdem wurde gefunden, dass eine Feststoffabtrennung vor der Extraktion in manchen Fällen die unerwünschte Mulmbildung nochmals deutlich vermindert oder vollständig unterdrückt.

Bevorzugt wird die Feststoffabtrennung derart ausgestattet, dass Feststoffpartikel mit einem hydraulischen Durchmesser größer 5 µm, insbesondere größer 1 µm und besonders bevorzugt größer 100 nm, abgetrennt werden.

Zur Feststoffabtrennung kann man übliche Verfahren verwenden, beispielsweise Filtration, Querstromfiltration, Zentrifugation, Sedimentation, Klassierung oder bevorzugt Dekantieren, wozu gängige Vorrichtungen wie Filter, Zentrifugen bzw. Dekanter verwendet werden können.

Temperatur und Druck bei der Feststoffabtrennung sind üblicherweise nicht kritisch. Beispielsweise kann man in den zuvor bzw. weiter unten genannten Temperatur- bzw. Druckbereichen arbeiten.

Die Feststoffabtrennung kann vor, während oder nach der - optionalen - Behandlung des Reaktionsaustrages bzw. des Stromes I mit Ammoniak bzw. Amin erfolgen. Dabei ist die Abtrennung während oder nach der Ammoniak- bzw. Aminbehandlung bevorzugt, und danach besonders bevorzugt.

Sofern man die Feststoffe während oder nach der Ammoniak- bzw. Aminbehandlung abtrennt, handelt es sich bei den Feststoffen zumeist um im Reaktionsaustrag schwerlösliche Verbindungen von Ammoniak bzw. Amin mit der verwendeten Lewis-Säure bzw. dem Promotor. Verwendet man beispielsweise ZnCl₂, so fällt bei der Ammoniakbehandlung im Wesentlichen schwerlösliches ZnCl₂· 2 NH₃ aus.

Sofern man die Feststoffe vor der Ammoniak- bzw. Aminbehandlung abtrennt oder falls gar keine Behandlung mit Ammoniak oder Amin erfolgt, handelt es sich bei den Feststoffen in der Regel um Nickelverbindungen der Oxidationsstufe +II, beispielsweise Nickel(II)cyanid oder ähnliche cyanidhaltige Nickel(II)verbindungen, oder um LewisSäuren bzw. deren Verbindungen. Die genannten Verbindungen können beispielsweise ausfallen, weil ihre Löslichkeit z.B. durch Temperaturänderung vermindert wurde.

### Optionale Verweilzeitstrecke

Der Strom I als Austrag von Schritt b) kann unmittelbar in Schritt c) überführt werden, beispielsweise durch eine Rohrleitung. Dabei bedeutet unmittelbar, dass die mittlere Verweilzeit von Strom I in der Rohrleitung weniger als 1 min beträgt.

Jedoch ist in einer bevorzugten Ausführungsform das erfindungsgemäße Verfahren dadurch gekennzeichnet, dass der Strom I nach Schritt b) und vor Schritt c) durch eine Verweilzeitstrecke geführt wird. Die Verweilzeitstrecke befindet sich folglich nach der Zugabe des Kohlenwasserstoffs K und vor der Extraktion.

Als Verweilzeitstrecke eignen sich z.B. Rohrleitungen, statische Mischer, gerührte oder ungerührte Behälter oder Behälterkaskaden, sowie Kombinationen dieser Elemente. Die Verweilzeitstrecke wird vorzugsweise derart bemessen und ausgestaltet, dass die mittlere Verweilzeit des Stroms I in der Verweilzeitstrecke mindestens 1 min, bevorzugt mindestens 5 min beträgt.

Die weiter oben beschriebene optionale Feststoffabtrennung kann auch in der Verweilzeitstrecke erfolgen. Dabei dient die Verweilzeitstrecke als Beruhigungszone, in der sich der Feststoff absetzen kann. Auf diese Weise wirkt die Verweilzeitstrecke wie ein Dekanter oder Querstromfilter. Sie kann mit Vorrichtungen zur Förderung und/oder zum Austrag von Feststoffen versehen sein.

Wie erwähnt wird in einer bevorzugten Ausführungsform der Kohlenwasserstoff K direkt in die Verweilzeitstrecke dosiert, beispielsweise an deren Anfang. Besonders bevorzugt wählt man in dieser Ausführungsform eine Verweilzeitstrecke, die eine innige Durchmischung von eingeengtem Reaktionsaustrag und Kohlenwasserstoff K gewährleistet.

Die Verweilzeitstrecke wird in der Regel bei Temperaturen von 0 bis 200, bevorzugt 10 bis 150 und insbesondere 20 bis 100°C, und Drücken von 0,01 bis 100, bevorzugt 0,1 bis 10 und insbesondere 0,5 bis 5 bar (abs.), betrieben.

In einer bevorzugten Ausgestaltung der Erfindung beträgt die Strömungsgeschwindigkeit des Stroms I in allen in dem erfindungsgemäßen Verfahren verwendeten Rohrleitungen mindestens 0,5, insbesondere mindestens 1 und besonders bevorzugt mindestens 2 m/s.

Der in Schritt b) erhaltene Strom I wird, ggf. nach der Behandlung mit Ammoniak oder Aminen, und/oder nach der Feststoffabtrennung und/oder nach Durchlaufen der Verweilzeitstrecke, in Schritt c) extrahiert.

### Schritt c): Extraktion des Stroms I mit dem Kohlenwasserstoff K

In Schritt c) wird der in Schritt b) erhaltene Strom I bei einer Temperatur T mit einem Kohlenwasserstoff K extrahiert; dabei erhält man einen Strom II enthaltend den mit dem Katalysator angereicherten Kohlenwasserstoff K und einen katalysatorarmen Strom III. Es bildet sich eine erste Phase (Strom II), die gegenüber dem Reaktionsaustrag am Katalysator angereichert ist, und eine zweite Phase (Strom III), die gegenüber dem Reaktionsaustrag an Dinitrilen angereichert ist. Zumeist ist Strom II die leichtere Phase, also die Oberphase, und Strom III die schwerere Phase, also die Unterphase.

Die Extraktion weist bevorzugt je nach Phasenverhältnis einen Extraktionskoeffizienten - definiert als Verhältnis aus dem Massengehalt an Katalysator, z.B. den genannten Nickel(0)-Komplexen bzw. Liganden, in Strom II zum Massengehalt an Katalyastor in Strom III -für jede theoretische Extraktionsstufe, von 0,1 bis 10, besonders bevorzugt 0,8 bis 5, auf. Dabei ist die Extraktionswirkung, gemessen am Extraktionskoeffizienten, für den freien Liganden gleich gut oder besser, bevorzugt besser als für den Nickel(0)-Komplex.

Strom II enthält nach der Phasentrennung vorzugsweise zwischen 50 und 99 Gew.-%, besonders bevorzugt zwischen 60 und 97 Gew.-%, insbesondere zwischen 80 und 95 Gew.-%, des zur Extraktion - ohne Berücksichtigung der zugefügten unpolaren aprotischen Flüssigkeit F - eingesetzten Kohlenwasserstoffes.

Die Lewis-Säure, die gegebenenfalls, nämlich bei der eingangs erwähnten zweiten Hydrocyanierung, im Zulaufstrom der Extraktion (Strom 1) enthalten ist, verbleibt vorzugsweise größtenteils und besonders bevorzugt vollständig in der Unterphase (Strom III). Hier bedeutet vollständig, dass die Restkonzentration der Lewis-Säure in der Oberphase (Strom II) vorzugsweise kleiner als 1 Gew.-%, besonders bevorzugt kleiner als 0,5 Gew.-%, insbesondere kleiner als 500 ppm by weight ist.

### Kohlenwasserstoff K

Der Kohlenwasserstoff ist das Extraktionsmittel. Er weist bevorzugt einen Siedepunkt von mindestens 30, besonders bevorzugt mindestens 60, insbesondere mindestens 90°C, und bevorzugt höchstens 140, besonders bevorzugt höchstens 135, insbesondere höchstens 130°C auf, jeweils bei einem Druck von 10⁵ Pa absolut.

Besonders bevorzugt kann ein Kohlenwasserstoff, wobei im Sinne der vorliegenden Erfindung hierunter ein einzelner Kohlenwasserstoff, wie auch ein Gemisch solcher Kohlenwasserstoffe verstanden wird, zur Abtrennung, insbesondere durch Extraktion, von Adipodinitril aus einer Mischung, enthaltend Adipodinitril und den (z.B. Ni(0) enthaltenden) Katalysator, eingesetzt werden, der einen Siedepunkt im Bereich zwischen 90°C und 140°C aufweist. Aus der nach der Abtrennung gemäß diesem Verfahren erhaltenen Mischung kann der Katalysator - ggf. unter Zusatz eines geeigneten Lösungsmittels, das höhersiedend ist als der Kohlenwasserstoff K (z.B. Pentennitril) - vorteilhaft durch destillative Abtrennung des Kohlenwasserstoffs erhalten werden. Dabei gestattet der Einsatz eines Kohlenwasserstoffs mit einem Siedepunkt in dem genannten Bereich eine besonders wirtschaftliche und technisch einfache Abtrennung, da man den abdestillierten Kohlenwasserstoff mit Flusswasser kondensieren kann.

Geeignete Kohlenwasserstoffe sind beispielsweise in US 3,773,809, Spalte 3, Zeile 50-62, beschrieben. Vorzugsweise kommt ein Kohlenwasserstoff, ausgewählt aus Cyclohexan, Methylcyclohexan, Cycloheptan, n-Hexan, n-Heptan, isomeren Heptanen, n-Octan, iso-Octan, isomeren Octanen wie 2,2,4-Trimethylpentan, cis- und trans-Decalin oder deren Gemische, insbesondere aus Cyclohexan, Methylcyclohexan, n-Heptan, isomeren Heptanen, n-Octan, isomeren Octanen wie 2,2,4-Trimethylpentan, oder deren Gemische, in Betracht. Besonders bevorzugt verwendet man Cyclohexan, Methylcyclohexan, n-Heptan oder n-Octan.

Ganz besonders bevorzugt sind n-Heptan oder n-Octan. Bei diesen Kohlenwasserstoffen ist die unerwünschte Mulmbildung besonders gering.

Der verwendete Kohlenwasserstoff ist vorzugsweise wasserfrei, wobei wasserfrei einen Wassergehalt von unter 100, vorzugsweise unter 50, insbesondere unter 10 ppm by weight bedeutet. Der Kohlenwasserstoff kann durch geeignete, dem Fachmann bekannte Verfahren getrocknet werden, beispielsweise durch Adsorption oder Azeotropdestillation. Die Trocknung kann in einem dem erfindungsgemäßen Verfahren vorgeschalteten Schritt erfolgen.

### Ausgestaltung der Extraktion

Die Extraktion des Katalysators, beispielsweise der Nickel(0)-Komplexe bzw. Liganden aus Strom I kann in jeder geeigneten, dem Fachmann bekannten Vorrichtung durchgeführt werden, bevorzugt in Gegenstrom-Extraktionskolonnen, Mixer-Settler-Kaskaden oder Kombinationen von Mixer-Settlem mit Kolonnen. Besonders bevorzugt ist die Verwendung von Gegenstrom-Extraktionskolonnen, die insbesondere mit Blechpackungen als dispergierende Elemente ausgestattet sind. In einer weiteren besonders bevorzugten Ausführungsform wird die Extraktion im Gegenstrom in einer kompartimentierten, gerührten Extraktionskolonne ausgeführt.

Betreffend die Dispergierrichtung wird in einer bevorzugten Ausführungsform des Verfahrens der Kohlenwasserstoff K als kontinuierliche Phase und Strom I als disperse Phase eingesetzt. Dies verkürzt in der Regel die Phasentrennzeit und vermindert nochmals die Mulmbildung. Jedoch ist auch die umgekehrte Dispergierrichtung möglich, also Strom I als kontinuierliche und Kohlenwasserstoff als disperse Phase, insbesondere dann, wenn die Mulmbildung vollständig unterdrückt werden kann. Üblicherweise wählt man die für die Trennleistung der Extraktionsvorrichtung günstigere Dispergierrichtung.

In der Extraktion wird ein Phasenverhältnis von vorzugsweise 0,1 bis 10, besonders bevorzugt 0,4 bis 2,5, insbesondere 0,75 bis 1,5, jeweils berechnet als Verhältnis von Masse des zugeführten Kohlenwasserstoffs K zu Masse von Strom I, verwendet.

Der absolute Druck während der Extraktion beträgt vorzugsweise 10 kPa bis 1 MPa, besonders bevorzugt 50 kPa bis 0,5 MPa, insbesondere 75 kPa bis 0,25 MPa (absolut).

Die Extraktion wird vorzugsweise bei einer Temperatur T von -15 bis 120, insbesondere 20 bis 100 und besonders bevorzugt 30 bis 80°C durchgeführt. Es wurde gefunden, dass bei höherer Temperatur der Extraktion die Mulmbildung geringer ist.

In einer besonders bevorzugten Ausführungsform wird die Extraktion mit einem Temperaturprofil betrieben. Insbesondere arbeitet man in diesem Fall bei einer Extraktionstemperatur von mindestens 60, bevorzugt 60 bis 95 und besonders bevorzugt mindestens 70°C.

Das Temperaturprofil ist bevorzugt derart ausgestaltet, dass in demjenigen Bereich der Extraktion, worin der Gehalt an Katalysator, z.B. an Nickel(0)-Komplexen mit phosphorhaltigen Liganden und/oder freien phosphorhaltigen Liganden, höher ist als im anderen Bereich, die Temperatur niedriger ist als im anderen Bereich. Auf diese Weise werden die temperaturlabilen Ni(0)-Komplexe thermisch weniger belastet und ihr Zerfall vermindert.

Verwendet man zur Extraktion beispielsweise eine Extraktionskolonne und wendet ein Temperaturprofil an, so wird am Kolonnenkopf die niedrigste und am Kolonnensumpf die höchste Temperatur eingestellt. Die Temperaturdifferenz zwischen Kolonnenkopf und -sumpf kann z.B. 0 bis 30, bevorzugt 10 bis 30 und insbesondere 20 bis 30°C betragen.

In einer besonders bevorzugten Ausführungsform wird der Strom I vor der Extraktion auf eine Temperatur T* temperiert, die mindestens 5, insbesondere mindestens 10 und besonders bevorzugt mindestens 20°C unter der eingeengten Temperatur T liegt, bei der die Extraktion erfolgt. Anstelle von Strom I kann man auch den eingeengten Reaktionsaustrag noch vor Schritt b), oder den zuzufügenden Kohlenwasserstoff K auf die genannte Temperatur T* temperieren. Die vorteilhafte Kühlung von Strom I kann also auch dadurch erfolgen, das man nicht erst den Strom I, sondern bereits vorab einen oder beide seiner Bestandteile (eingeengten Reaktionsaustrag oder Kohlenwasserstoff K) kühlt.

Demnach ist das Verfahren bevorzugt dadurch gekennzeichnet, dass mindestens einer der drei Ströme eingeengter Reaktionsaustrag, Kohlenwasserstoff K und Strom I, auf eine Temperatur T* temperiert wird, die mindestens 5°C, insbesondere mindestens 10°C und besonders bevorzugt mindestens 20°C unter der Temperatur T liegt. Besonders bevorzugt ist eine Temperaturdifferenz ΔT = T - T*, bei der eine Abscheidung von Feststoffen in der Extraktionsstufe c) vermieden wird.

In manchen Fällen kann es vorteilhaft sein, nach der Abkühlung auf die niedrigere Temperatur T* den eingeengten Reaktionsaustrag, den Kohlenwasserstoff K bzw. den Strom I vor dem Eintritt in die Extraktion wieder zu erwärmen, beispielsweise auf die Temperatur T.

### Ausgestaltung der Phasentrennung

Die Phasentrennung kann räumlich und zeitlich je nach apparativer Ausgestaltung auch als letzter Teil der Extraktion betrachtet werden. Zur Phasentrennung kann üblicherweise ein weiter Druck-, Konzentrations- und Temperaturbereich gewählt werden, wobei die für die jeweilige Zusammensetzung der Reaktionsmischung optimalen Parameter leicht durch wenige einfache Vorversuche ermittelt werden können.

Die Temperatur bei der Phasentrennung beträgt üblicherweise mindestens 0, vorzugsweise mindestens 10, besonders bevorzugt mindestens 20°C. Üblicherweise beträgt sie höchstens 120, vorzugsweise höchstens 100, besonders bevorzugt höchstens 95°C. Beispielsweise führt man die Phasentrennung bei 0 bis 100, bevorzugt 60 bis 95°C durch. Es wurde gefunden, dass bei höherer Temperatur der Phasentrennung die Mulmbildung geringer ist.

Der Druck bei der Phasentrennung liegt in der Regel bei mindestens 1 kPa, vorzugsweise mindestens 10 kPa, besonders bevorzugt 20 kPa. In der Regel beträgt er höchstens 2 MPa, vorzugsweise höchstens 1 MPa, besonders bevorzugt höchstens 0,5 MPa absolut.

Die Phasentrennzeit, also die Zeitspanne von der Vermischung des Stroms I mit dem Kohlenwasserstoff K (Extraktionsmittel) bis zur Ausbildung einer einheitlichen Oberphase und einer einheitlichen Unterphase, kann in weiten Grenzen variieren. Die Phasentrennzeit beträgt in der Regel 0,1 bis 60, bevorzugt 1 bis 30 und insbesondere 2 bis 10 min. Bei großtechnischer Durchführung des erfindungsgemäßen Verfahrens ist üblicherweise eine Phasentrennzeit von maximal 15, insbesondere maximal 10 min technisch und ökonomisch sinnvoll.

Es wurde gefunden, dass sich die Phasentrennzeit insbesondere bei Verwendung langkettiger aliphatischer Alkane wie n-Heptan oder n-Octan als Kohlenwasserstoff K in vorteilhafter Weise vermindert.

Die Phasentrennung kann in einer oder mehreren dem Fachmann für solche Phasentrennungen bekannten Vorrichtungen durchgeführt werden. In einer vorteilhaften Ausführungsform kann man die Phasentrennung in der Extraktionsvorrichtung durchführen, beispielsweise in einer oder mehreren Mixer-Settler-Kombinationen oder durch Ausstattung einer Extraktionskolonne mit einer Beruhigungszone.

Bei der Phasentrennung erhält man zwei flüssige Phasen, von denen eine Phase (Strom II) einen höheren Anteil an Katalysator, z.B. dem Nickel(0)-Komplex mit phosphorhaltigen Liganden und/oder freien phosphorhaltigen Liganden, bezogen auf das Gesamtgewicht dieser Phase, aufweist als die andere Phase (Strom III).

### Nicket(0)-Komptexe und Liganden

Bei den als Katalysator bevorzugt verwendeten Nickel(0)-Komplexen, die phosphorhaltige Liganden und/oder freie phosphorhaltige Liganden enthalten, handelt es sich bevorzugt um homogen gelöste Nickel(0)-Komplexe.

Die phosphorhaltigen Liganden der Nickel(0)-Komplexe und die freien phosphorhaltigen Liganden, die erfindungsgemäß durch Extraktion abgetrennt werden, sind vorzugsweise ausgewählt aus mono- oder bidentaten Phosphinen, Phosphiten, Phosphiniten und Phosphoniten.

Diese phosphorhaltigen Liganden weisen vorzugsweise die Formel I auf:

P (X¹R¹) (X²R²) (X³R³) (I)

Unter Verbindung I wird im Sinne der vorliegenden Erfindung eine einzelne Verbindung oder ein Gemisch verschiedener Verbindungen der vorgenannten Formel verstanden.

Erfindungsgemäß sind X¹, X², X³ unabhängig voneinander Sauerstoff oder Einzelbindung. Falls alle der Gruppen X¹, X² und X³ für Einzelbindungen stehen, so stellt Verbindung I ein Phosphin der Formel P(R¹R²R³) mit den für R¹, R² und R³ in dieser Beschreibung genannten Bedeutungen dar.

Falls zwei der Gruppen X¹, X² und X³ für Einzelbindungen stehen und eine für Sauerstoff, so stellt Verbindung I ein Phosphinit der Formel P(OR¹)(R²)(R³) oder P(R¹)(OR²)(R³) oder P(R¹)(R²)(OR³) mit den für R¹, R² und R³ weiter unten genannten Bedeutungen dar.

Falls eine der Gruppen X¹, X² und X³ für eine Einzelbindung steht und zwei für Sauerstoff, so stellt Verbindung I ein Phosphonit der Formel P(OR¹)(OR²)(R³) oder P(R¹)(OR²)(OR³) oder P(OR¹)(R²)(OR³) mit den für R¹, R² und R³ in dieser Beschreibung genannten Bedeutungen dar.

In einer bevorzugten Ausführungsform sollten alle der Gruppen X¹, X² und X³ für Sauerstoff stehen, so dass Verbindung I vorteilhaft ein Phosphit der Formel P(OR¹)(OR²)(OR³) mit den für R¹, R² und R³ weiter unten genannten Bedeutungen darstellt.

Erfindungsgemäß stehen R¹, R², R³ unabhängig voneinander für gleiche oder unterschiedliche organische Reste. Als R¹, R² und R³ kommen unabhängig voneinander Alkylreste, vorzugsweise mit 1 bis 10 Kohlenstoffatomen, wie Methyl, Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorzugsweise mit 1 bis 20 Kohlenstoffatomen, wie 1,1'-Biphenol, 1,1'-Binaphthol in Betracht. Die Gruppen R¹, R² und R³ können miteinander direkt, also nicht allein über das zentrale Phosphor-Atom, verbunden sein. Vorzugsweise sind die Gruppen R¹, R² und R³ nicht miteinander direkt verbunden.

In einer bevorzugten Ausführungsform kommen als Gruppen R¹, R² und R³ Reste ausgewählt aus der Gruppe bestehend aus Phenyl, o-Tolyl, m-Tolyl und p-Tolyl in Betracht. In einer besonders bevorzugten Ausführungsform sollten dabei maximal zwei der Gruppen R¹, R² und R³ Phenyl-Gruppen sein.

In einer anderen bevorzugten Ausführungsform sollten dabei maximal zwei der Gruppen R¹, R² und R³ o-Tolyl-Gruppen sein.

Als besonders bevorzugte Verbindungen I können solche der Formel I a

(o-Tolyl-O-)_{w} (m-Tolyl-O-)ₓ (p-Tolyl-O-)_{y} (Phenyl-O-)_{z} P (I a)

eingesetzt werden, wobei w, x, y und z eine natürliche Zahl bedeuten, und folgende Bedingungen gelten: w + x + y + z = 3 und w, z ≤2.

Solche Verbindungen I a sind z.B. (p-Tolyl-O-)(Phenyl-O-)₂P, (m-Tolyl-O-)(Phenyl-O-)₂P, (o-Tolyl-O-) (Phenyl-O-)₂P, (p-Tolyl-O-)₂(Phenyl-O-)P, (m-Tolyl-O-)₂(Phenyl-O-)P, (o-Tolyl-O-)₂(Phenyl-O-)P, (m-Tolyl-O-)(p-Tolyl-O)(Phenyl-O-)P, (o-Tolyl-O-)(p-Tolyl-O-)(Phenyl-O-)P, (o-Tolyl-O-)(m-Tolyl-O-)(Phenyl-O-)P, (p-Tolyl-O-)₃P, (m-Tolyl-O-)(p-Tolyl-O-)₂P, (o-Tolyl-O-)(p-Tolyl-O-)₂P, (m-Tolyl-O-)₂(p-Toluyl-O-)P, (o-Tolyl-O-)₂(p-Tolyl-O-)P, (o-Tolyl-O-)(m-Tolyl-O-)(p-Tolyl-O)P, (m-Tolyl-O-)₃P, (o-Tolyl-O-)(m-Tolyl-O-)₂P (o-Tolyl-O-)₂(m-Tolyl-O-)P, oder Gemische solcher Verbindungen.

Gemische enthaltend (m-Tolyl-O-)₃P, (m -Tolyl-O-)₂(p-Tolyl-O-)P, (m-Tolyl-O-)(p-Tolyl-O-)₂P und (p-Tolyl-O-)₃P kann man beispielsweise durch Umsetzung eines Gemisches enthaltend m-Kresol und p-Kresol, insbesondere im Molverhältnis 2 : 1, wie es bei der destillativen Aufarbeitung von Erdöl anfällt, mit einem Phosphortrihalogenid, wie Phosphortrichlorid, erhalten.

In einer anderen, ebenfalls bevorzugten Ausführungsform kommen als phosphorhaltige Liganden die in der DE-A 199 53 058 näher beschriebenen Phosphite der Formel I b in Betracht:

P (O-R¹)ₓ (O-R²)_{y} (O-R³)_{z} (O-R⁴)ₚ (I b)

mit
- R¹:: aromatischer Rest mit einem C₁-C₁₈-Alkylsubsfituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromatischen System verbindet, anellierten aromatischen System,
- R²:: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der aromatische Rest in o-Stellung zu dem Sauerstoffatom, das das Phosphoratom mit dem aromatischen System verbindet, ein Wasserstoffatom trägt,
- R³:: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt,
- R⁴:: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt,
- x :: 1 oder 2,
- y, z, p:: unabhängig voneinander 0, 1 oder 2 mit der Maßgabe, dass x+y+z+p = 3.

Bevorzugte Phosphite der Formel I b sind der DE-A 199 53 058 zu entnehmen. Als Rest R¹ kommen vorteilhaft o-Tolyl-, o-Ethyl-phenyl-, o-n-Propyl-phenyl-, o-Isopropyl-phenyl-, o-n-Butyl-phenyl-, o-sek-Butyl-phenyl-, o-tert-Butyl-phenyl-, (o-Phenyl)-Phenyl- oder 1-Naphthyl- Gruppen in Betracht.

Als Rest R² sind m-Tolyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen bevorzugt.

Als Rest R³ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Rest R⁴ ist bevorzugt Phenyl. Vorzugsweise ist p gleich null. Für die Indizes x, y, z und p in Verbindung I b ergeben sich folgende Möglichkeiten:

| x | y | z | p |
|---|---|---|---|
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

Bevorzugte Phosphite der Formel I b sind solche, in denen p gleich null ist sowie R¹, R² und R³ unabhängig voneinander ausgewählt sind aus o-Isopropyl-phenyl, m-Tolyl und p-Tolyl, und R⁴ Phenyl ist.

Besonders bevorzugte Phosphite der Formel I b sind solche, in denen R¹ der o-lsopropyl-phenyl-Rest, R² der m-Tolylrest und R³ der p-Tolylrest ist mit den in der vorstehenden Tabelle genannten Indizes; außerdem solche, in denen R¹ der o-Tolylrest, R² der m-Tolylrest und R³ der p-Tolylrest ist mit den in der Tabelle genannten Indizes; weiterhin solche, in denen R¹ der 1-Naphthylrest, R² der m-Tolylrest und R³ der p-Tolylrest ist mit den in der Tabelle genannten Indizes; außerdem solche, in denen R¹ der o-Tolylrest, R² der 2-Naphthylrest und R³ der p-Tolylrest ist mit den in der Tabelle genannten Indizes; und schließlich solche, in denen R¹ der o-Isopropyl-phenyl-Rest, R² der 2-Naphthylrest und R³ der p-Tolylrest ist mit den in der Tabelle genannten Indizes; sowie Gemische dieser Phosphite.

Phosphite der Formel I b können erhalten werden, indem man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹OH, R²OH, R³OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹OH, R²OH, R³OH und R⁴OH oder deren Gemische umsetzt unter Erhalt eines Phosphits der Formel I b.

Die Umsetzung kann in drei getrennten Schritten durchgeführt werden. Ebenso können zwei der drei Schritte kombiniert werden, also a) mit b) oder b) mit c). Alternativ können alle der Schritte a), b) und c) miteinander kombiniert werden.

Dabei kann man geeignete Parameter und Mengen der Alkohole ausgewählt aus der Gruppe bestehend aus R¹OH, R²OH, R³OH und R⁴OH oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere Cl, eingesetzt wird, sowie deren Gemische in Betracht. Es können auch Gemische verschiedener gleich oder unterschiedlich halogensubstituierter Phosphine als Phosphortrihalogenid eingesetzt werden. Besonders bevorzugt ist PCl₃. Weitere Einzelheiten zu den Reaktionsbedingungen bei der Herstellung der Phosphite I b und zur Aufarbeitung sind der DE-A 199 53 058 zu entnehmen.

Die Phosphite I b können auch in Form eines Gemisches verschiedener Phosphite I b als Ligand verwendet werden. Ein solches Gemisch kann beispielsweise bei der Herstellung der Phosphite I b anfallen.

Es ist allerdings bevorzugt, dass der phosphorhaltige Ligand mehrzähnig, insbesondere zweizähnig ist. Daher weist der verwendete Ligand vorzugsweise die Formel II 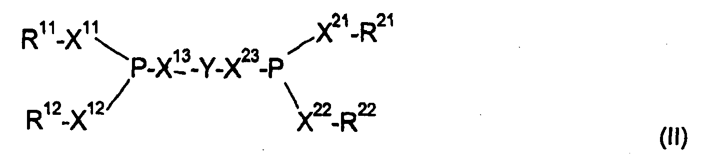 auf, worin bedeuten
- X¹¹, X¹², X¹³, X²¹, X²², X²³: unabhängig voneinander Sauerstoff oder Einzelbindung
- R¹¹, R¹²: unabhängig voneinander gleiche oder unterschiedliche, einzelne oder verbrückte organische Reste
- R²¹, R²²: unabhängig voneinander gleiche oder unterschiedliche, einzelne oder verbrückte organische Reste,
- Y: Brückengruppe

Unter Verbindung II wird im Sinne der vorliegenden Erfindung eine einzelne Verbindung oder ein Gemisch verschiedener Verbindungen der vorgenannten Formel verstanden.

In einer bevorzugten Ausführungsform können X¹¹, X¹², X¹³, X²¹, X²², X²³ Sauerstoff darstellen. In einem solchen Fall ist die Brückengruppe Y mit Phosphit-Gruppen verknüpft.

In einer anderen bevorzugten Ausführungsform können X¹¹ und X¹² Sauerstoff und X¹³ eine Einzelbindung oder X¹¹ und X¹³ Sauerstoff und X¹² eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen bevorzugten Ausführungsform können X¹³ Sauerstoff und X¹¹ und X¹² eine Einzelbindung oder X¹¹ Sauerstoff und X¹² und X¹³ eine Einzelbindung darstellen, so dass das mit X¹¹, X¹² und X¹³ umgebene Phosphoratom Zentralatom eines Phosphinits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphinits oder Phosphins, vorzugsweise eines Phosphinits, sein kann.

In einer anderen bevorzugten Ausführungsform können X¹¹, X¹² und X¹³ eine Einzelbindung darstellen, so dass das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so dass das mit X²¹, X²² und X²³ umgebene Phosphoratom Zentralatom eines Phosphits oder Phosphins, vorzugsweise eines Phosphins, sein kann.

Als Brückengruppe Y kommen vorzugsweise substituierte, beispielsweise mit C₁-C₄-Alkyl, Halogen, wie Fluor, Chlor, Brom, halogeniertem Alkyl, wie Trifluormethyl, Aryl, wie Phenyl, oder unsubstituerte Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterscheidliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R¹¹ und R¹² können einzeln oder verbrückt sein. Auch die Reste R²¹ und R²² können einzeln oder verbrückt sein. Die Reste R¹¹, R¹², R²¹ und R²² können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht. In einer besonders bevorzugten Ausführungsform kommen die in US 5,512,696 genannten Verbindungen der Formel I, II, III IV, V, VI und VII, insbesondere die dort in den Beispielen 1 bis 31 eingesetzten Verbindungen, in Betracht. In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzten Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzten Verbindungen, in Betracht. In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzten Verbindungen, in Betracht.

In einer besonders bevorzugten Ausführungsform kommen die in US 6,127,567 genannten Verbindungen und dort in den Beispielen 1 bis 29 eingesetzten Verbindungen in Betracht. In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzten Verbindungen, in Betracht. In einer besonders bevorzugten Ausführungsform kommen die in US 5,959,135 genannten Verbindungen und dort in den Beispielen 1 bis 13 eingesetzten Verbindungen in Betracht.

In einer besonders bevorzugten Ausführungsform kommen die in US 5,847,191 genannten Verbindungen der Formel I, II und III in Betracht. In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 13, 14, 15, 16, 17, 18, 19, 20 und 21 dargestellten Verbindungen, in Betracht. In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht. In einer besonders bevorzugten Ausführungsform kommen die in WO 99/13983 genannten Verbindungen in Betracht. In einer besonders bevorzugten Ausführungsform kommen die in WO 99/64155 genannten Verbindungen in Betracht.

In einer besonders bevorzugten Ausführungsform kommen die in der deutschen Patentanmeldung DE 100 380 37 genannten Verbindungen in Betracht. In einer besonders bevorzugten Ausführungsform kommen die in der deutschen Patentanmeldung DE 100 460 25 genannten Verbindungen in Betracht. In einer besonders bevorzugten Ausführungsform kommen die in der deutschen Patentanmeldung DE 101 502 85 genannten Verbindungen in Betracht.

In einer besonders bevorzugten Ausführungsform kommen die in der deutschen Patentanmeldung DE 101 502 86 genannten Verbindungen in Betracht. In einer besonders bevorzugten Ausführungsform kommen die in der deutschen Patentanmeldung DE 102 071 65 genannten Verbindungen in Betracht. In einer weiteren besonders bevorzugten Ausführungsform der vorliegenden Erfindung kommen die in der US 2003/0100442 A1 genannten phosphorhaltigen Chelatliganden in Betracht.

In einer weiteren besonders bevorzugten Ausführungsform der vorliegenden Erfindung kommen die in der nicht vorveröffentlichten deutschen Patentanmeldung Aktenzeichen DE 103 50 999.2 vom 30.10.2003 genannten phosphorhaltigen Chelatliganden in Betracht.

Die beschriebenen Verbindungen I, I a, I b und II sowie deren Herstellung sind an sich bekannt. Als phosphorhaltiger Ligand können auch Mischungen, enthaltend mindestens zwei der Verbindungen I, I a, I b und II, eingesetzt werden.

In einer besonders bevorzugten Ausführungsform des erfindungsgemäßen Verfahrens ist der phosphorhaltige Ligand des Nickel(0)-Komplexes und/oder der freie phosphorhaltige Ligand ausgewählt aus Tritolylphosphit, bidentaten phosphorhaltigen Chelatliganden, sowie den Phosphiten der Formel I b

P (O-R¹)ₓ (O-R²)_{y} (O-R³)_{z} (O-R⁴)ₚ (I b)

worin R¹, R² und R³ unabhängig voneinander ausgewählt sind aus o-Isopropyl-phenyl, m-Tolyl und p-Tolyl, R⁴ Phenyl ist; x gleich 1 oder 2 ist, und y, z, p unabhängig voneinander 0, 1 oder 2 sind mit der Maßgabe, dass x+y+z+p = 3 ist; und deren Mischungen.

### Lewis-Säure bzw. Promotor

Im Sinne der vorliegenden Erfindung wird unter einer Lewis-Säure eine einzelne Lewis-Säure, wie auch ein Gemisch aus mehreren, wie zwei, drei oder vier Lewis-Säuren, verstanden.

Als Lewis-Säure kommen dabei anorganische oder organische Metall-Verbindungen in Betracht, in denen das Kation ausgewählt ist aus der Gruppe bestehend aus Scandium, Titan, Vanadium, Chrom, Mangan, Eisen, Kobalt, Kupfer, Zink, Bor, Aluminium, Yttrium, Zirkonium, Niob, Molybdän, Cadmium, Rhenium und Zinn. Beispiele umfassen ZnBr₂, Znl₂, ZnCl₂, ZnSO₄, CuCl₂, CuCl, Cu(O₃SCF₃)₂, CoCl₂, Col₂, Fel₂, FeCl₃, FeCl₂, FeCl₂(THF)₂, TiCl₄(THF)₂, TiCl₄, TiCl₃, ClTi(O-i-Propyl)₃, MnCl₂, ScCl₃, AlCl₃, (C₈H₁₇)AlCl₂, (C₈H₁₇)₂AlCl, (i-C₄H₈)₂AlCl, (C₆H₅)₂AlCl, (C₆H₅)AlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆H₅)₃, TaCl₅, wie beispielsweise in US 6,127,567, US 6,171,996 und US 6,380,421 beschrieben. Weiterhin kommen in Betracht Metallsalze, wie ZnCl₂, Col₂ und SnCl₂ und organometallische Verbindungen, wie RAlCl₂, R₂AlCl, RSnO₃SCF₃ und R₃B, wobei R eine Alkyl- oder Aryl-Gruppe ist, wie beispielsweise in US 3,496,217, US 3,496,218 und US 4,774,353 beschrieben.

Weiterhin können gemäß US 3,773,809 als Promotor ein Metall in kationischer Form, ausgewählt aus der Gruppe bestehend aus Zink, Cadmium, Beryllium, Aluminium, Gallium, Indium, Thallium, Titan, Zirkonium, Hafnium, Erbium, Germanium, Zinn, Vanadium, Niob, Scandium, Chrom, Molybdän, Wolfram, Mangan, Rhenium, Palladium, Thorium, Eisen und Kobalt, vorzugsweise Zink, Cadmium, Titan, Zinn, Chrom, Eisen und Kobalt, eingesetzt werden, wobei der anionische Teil der Verbindung ausgewählt sein kann aus der Gruppe bestehend aus Halogeniden, wie Fluorid, Chlorid, Bromid und Jodid, Anionen niedriger Fettsäuren mit von 2 bis 7 Kohlenstoffatomen, HPO₃²⁻, H₃PO²⁻, CF₃COO⁻, C₇H₁₅OSO₂⁻ oder SO₄²⁻. Weiterhin sind aus US 3,773,809 als geeignete Promotoren Borhydride, Organoborhydride und Borsäureester der Formel R₃B und B(OR)₃, wobei R ausgewählt ist aus der Gruppe bestehend aus Wasserstoff, Aryl-Radikale mit zwischen 6 und 18 Kohlenstoff-Atomen, mit Alkyl-Gruppen mit 1 bis 7 Kohlenstoff-Atomen substituierte Aryl-Radikale und mit Cyano-substituierte Alkyl-Gruppen mit 1 bis 7 Kohlenstoff-Atomen substituierte Aryl-Radikale, vorteilhaft Triphenylbor, genannt.

Weiterhin können, wie in US 4,874,884 beschrieben, synergistisch wirksame Kombinationen von Lewis-Säuren eingesetzt werden, um die Aktivität des Katalysatorsystems zu erhöhen. Geeignete Promotoren können beispielsweise aus der Gruppe bestehend aus CdCl₂, FeCl₂, ZnCl₂, B(C₆H₅)₃ und (C₆H₅)₃SnX, mit X gleich CF₃SO₃, CH₃C₆H₄SO₃ oder (C₆H₅)₃BCN ausgewählt werden, wobei für das Verhältnis von Promotor zu Nickel ein Bereich von vorzugsweise etwa 1:16 bis etwa 50:1 genannt ist.

Im Sinne der vorliegenden Erfindung umfasst der Begriff Lewis-Säure auch die in US 3,496,217, US 3,496,218, US 4,774,353, US 4,874,884, US 6,127,567, US 6,171,996 und US 6,380,421 genannten Promotoren.

Als besonders bevorzugte Lewis-Säuren kommen unter den genannten insbesondere Metallsalze, besonders bevorzugt Metallhalogenide, wie Fluoride, Chloride, Bromide, Jodide, insbesondere Chloride, in Betracht, von denen wiederum Zinkchlorid, Eisen-(II)-chlorid und Eisen-(III)-chlorid besonders bevorzugt sind.

Mit dem erfindungsgemäßen Verfahren wird die unerwünschte Mulmbildung deutlich vermindert oder unterbleibt ganz. Die Extraktionsvorrichtung wird nicht mit Mulm geflutet und kann auch über lange Zeit störungsfrei betrieben werden. Die Wiederfindung des Katalysators in der Kohlenwasserstoffphase (Strom II) ist ebenso gut wie in den Verfahren des Standes der Technik.

Durch die optionale Behandlung mit Ammoniak bzw. Aminen, die optionale Abtrennung der Feststoffes sowie die optionale Verweilzeitstrecke, lässt sich das Verfahren weiter optimieren, die Mulmbildung weiter reduzieren und die Trennleistung der Extraktion einstellen.

### Beispiele

Als Kohlenwasserstoff K (Extraktionsmittel) wurde n-Heptan verwendet.

Der eingesetzte Reaktionsaustrag der Hydrocyanierung von Pentennitril zu Adipodinitril wies folgende Zusammensetzung auf:
- 29,5 Gew.-% C₆-Dinitrile: u.a. Adipodinitril, 2-Methylglutarsäuredinitril, Ethylbernsteinsäuredinitril,
- 51,3 Gew.-% C₅-Mononitrile: überwiegend trans-3-Pentennitril, außerdem weitere lineare Pentennitrilisomere, und
- 19,2 Gew.-% Katalysatorkomponenten: u.a. Ni(0)-Tritolylphosphit-Komplexe, freie Tritolylphosphit-Liganden, Zinkchlorid und Abbauprodukte des Katalysators beziehungsweise des Tritolylphosphits.

Nicht erfindungsgemäßes Beispiel A: keine Zugabe von Heptan vor der Extraktion

Der mit einem Massestrom von 78 kg/h anfallende Reaktionsaustrag wurde durch Destillation bei einem absoluten Druck von 5 mbar in einem umgepumpten Behälter um 38 kg/h auf 40 kg/h eingeengt. Man erhielt einen eingeengten Austrag folgender Zusammensetzung:
- 57,5 Gew.-% C₆-Dinitrile,
- 5 Gew.-% C₆-Mononitrile, und
- 37,5 Gew.-% Katalysatorkomponenten.

Dieser eingeengte Austrag wurde in einer Gegenstrom-Extraktionskolonne mit einer Blechpackung umfassend 7 theoretische Trennstufen und einem Innendurchmesser von 100 mm im Gegenstrom mit 120 kg/h n-Heptan extrahiert. Dabei wählte man die Dispergierrichtung derart, dass n-Heptan die kontinuierliche Phase darstellt. Die Kolonne wurde durch eine Doppelmantelbeheizung mit Warmwasser von 70 °C Vorlauftemperatur temperiert.

Die Kolonne zeigte erhebliche Mulmbildung, und nach ca. 17 h Betriebszeit war sie vollständig mit Mulm gefüllt. Die Extraktion musste abgebrochen werden, weil sie so nicht mehr zu betreiben war.

### Erfindungsgemäßes Beispiel 1: Zugabe von Heptan vor der Extraktion

Man engte den Reaktionsaustrag ein wie in Beispiel A beschrieben. Der eingeengte Austrag (Zusammensetzung siehe Beispiel A) wurde in einem Umpumpkreislauf mit 20 kg/h n-Heptan vermischt. Der Umpumpkreislauf bestand aus ca. 10 m Rohrleitung mit einem Innendurchmesser von 15 mm und einer Zahnradpumpe als Umlaufpumpe, die gegen einen Überströmer arbeitete, der bei 5 bar Überdruck öffnete. Die Pumpe förderte einen Volumenstrom von 500 l/h.

Nach dem Austritt aus dem Umpumpkreislauf wurde der erhaltene Strom I in einen 50 I-Glasbehälter (als Verweilzeitstrecke) geleitet. Aus einem Überlauf des Glasbehälters trat ein Strom I aus, den man dem unteren Ende einer Extraktionskolonne zuführte. Die Extraktionskolonne und deren Betriebsbedingungen waren identisch mit Beispiel A mit der Ausnahme, dass am Kopf der Kolonne 100 kg/h n-Heptan zugefahren wurden.

Die Kolonne zeigte auch nach einer Betriebsdauer von 11 Tagen keine Mulmbildung und konnte störungsfrei betrieben werden. Es wurde eine Heptanphase (Strom II) und eine Nitrilphase (Strom III) abgezogen.

Um die Wirksamkeit der Extraktion zu beurteilen, wurden die Heptanphase und die Nitrilphase mittels Cyclovoltametrie auf Ni(0) und mittels Atomabsorptionsspektroskopie auf Phosphor untersucht. Die Heptanphase enthielt 99,4 % des Ni(0) und 99,2 % des Phosphors aus dem Zulauf der Extraktion; in der Nitrilphase war kein Ni(0) und 0,7 % des Phosphors nachweisbar.

Die Beispiele zeigen, dass bei dem erfindungsgemäßen Verfahren die unerwünschte Mulmbildung vollständig unterblieb. Dabei wurde die Wirksamkeit der Extraktion nicht beeinträchtigt.

## Patentansprüche

1. Verfahren zur extraktiven Abtrennung von homogen gelösten Katalysatoren aus einem Reaktionsaustrag einer Hydrocyanierung von ungesättigten Mononitrilen zu Dinitrilen, durch Extraktion mittels eines Kohlenwasserstoffs K, **dadurch gekennzeichnet, dass** man
a) den Reaktionsaustrag vor Schritt b) durch Destillation bei Drücken von 0,1 bis 5000 mbar und Temperatur von 10 bis 150°C einengt,
b) dem eingeengten Reaktionsaustrag einen Kohlenwasserstoff K zufügt, wobei ein Strom I erhalten wird, und
c) den Strom I ohne vorherige Trennung der flüssigen Phasen in eine Extraktionsvorrichtung einspeist und bei einer Temperatur T mit dem Kohlenwasserstoff K extrahiert, wobei ein Strom II enthaltend den mit dem Katalysator angereicherten Kohlenwasserstoff K, und ein katalysatorarmer Strom III erhalten wird.

2. Verfahren nach Anspruch 1, **dadurch gekennzeichnet, dass** man den eingeengten Reaktionsaustrag vor Schritt b), bzw. den Strom I während oder nach Schritt b) oder während Schritt c), mit Ammoniak oder einem primären, sekundären oder tertiären aromatischen oder aliphatischen Amin behandelt.

3. Verfahren nach den Ansprüchen 1 bis 2, **dadurch gekennzeichnet, dass** man den Ammoniak bzw. das Amin zusammen mit Kohlenwasserstoff K zufügt.

4. Verfahren nach den Ansprüchen 1 bis 3, **dadurch gekennzeichnet, dass** in Schritt b) ausfallende Feststoffe vor Schritt c) aus dem Strom I abgetrennt werden.

5. Verfahren nach den Ansprüchen 1 bis 4, **dadurch gekennzeichnet, dass** man die Feststoffabtrennung vor, während oder nach der Behandlung des Reaktionsaustrages bzw. Strom I durchführt.

6. Verfahren nach den Ansprüchen 1 bis 5, **dadurch gekennzeichnet, dass** es sich beim Katalysator um Nickel(O)-Komplexe mit phosphorhaltigen Liganden, und/oder freie phosphorhaltige Liganden, handelt.

7. Verfahren nach den Ansprüchen 1 bis 6, **dadurch gekennzeichnet, dass** das Mononitril 3-Pentennitril und das Dinitril Adipodinitril ist.

8. Verfahren nach den Ansprüchen 1 bis 7, **dadurch gekennzeichnet, dass** man den Reaktionsaustrag durch Umsetzung von 3-Pentennitril mit Cyanwasserstoff in Gegenwart mindestens eines Nickel(O)-Komplexes mit phosphorhaltigen Liganden, gegebenenfalls in Gegenwart mindestens einer Lewis-Säure, erhält.

9. Verfahren nach den Ansprüchen 1 bis 8, **dadurch gekennzeichnet, dass** man als Kohlenwasserstoff K Cyclohexan, Methylcyclohexan, n-Heptan oder n-Octan verwendet.

10. Verfahren nach den Ansprüchen 1 bis 9, **dadurch gekennzeichnet, dass** man in Schritt b) den Kohlenwasserstoff K mit dem eingeengten Reaktionsaustrag in einem Rührbehälter oder einem Umpumpkreislauf vermischt.

11. Verfahren nach den Ansprüchen 1 bis 10, **dadurch gekennzeichnet, dass** der Kohlenwassertoff K ein Teilstrom des Stroms II ist.

12. Verfahren nach den Ansprüchen 1 bis 11, **dadurch gekennzeichnet** das die Extraktion in Schritt c) in einer Gegenstromextraktionskolonne durchgeführt wird, die mit Blechpackungen als dispergierenden Elemente ausgestattet ist.

13. Verfahren nach den Ansprüchen 1 bis 12, **dadurch gekennzeichnet** das die Extraktion in Schritt c) im Gegenstrom in einer kompaitimentierten, gerührten Extraktionskolonne durchgeführt wird.

14. Verfahren nach den Ansprüchen 1 bis 13, **dadurch gekennzeichnet, dass** der Strom I nach Schritt b) und vor Schritt c) durch eine Verweilzeitstrecke geführt wird.

15. Verfahren nach den Ansprüchen 1 bis 14, **dadurch gekennzeichnet, dass** die mittlere Verweilzeit des Stroms I in der Verweilzeitstrecke mindestens 1 min beträgt.

16. Verfahren nach den Ansprüchen 1 bis 15, **dadurch gekennzeichnet, dass** die Strömungsgeschwindigkeit des Stroms I in allen in dem Verfahren verwendeten Rohrleitungen mindestens 2 m/s beträgt.

17. Verfahren nach den Ansprüchen 1 bis 16, **dadurch gekennzeichnet, dass** mindestens einer der drei Ströme eingeengter Reaktionsaustrag, Kohlenwasserstoff K und Strom I, auf eine Temperatur T* temperiert wird, die mindestens 5°C unter der Temperatur T liegt.

## Claims

1. A process for extractively removing homogeneously dissolved catalysts from a reaction effluent of a hydrocyanation of unsaturated mononitriles to dinitriles, by extraction by means of a hydrocarbon H, which comprises
a) concentrating the reaction effluent before step b) by distillation at pressures of from 0.1 to 5000 mbar and temperatures of from 10 to 150°C,
b) adding a hydrocarbon H to the concentrated reaction effluent to obtain a stream I, and
c) feeding stream I, without prior separation of the liquid phases, into an extraction apparatus and extracting it at a temperature T with the hydrocarbon H to obtain a stream II comprising the hydrocarbon H enriched with the catalyst and a stream III having a low catalyst content.

2. The process according to claim 1, wherein the concentrated reaction effluent is treated before step b), or stream I is treated during or after step b) or during step c), with ammonia or a primary, secondary or tertiary, aromatic or aliphatic amine.

3. The process according to claims 1 and 2, wherein the ammonia or the amine is added together with hydrocarbon H.

4. The process according to claims 1 to 3, wherein solids precipitating in step b) are removed from stream I before step c).

5. The process according to claims 1 to 4, wherein the solids removal is carried out before, during or after the treatment of the reaction effluent or of stream I.

6. The process according to claims 1 to 5, wherein the catalyst is a nickel(0) complex having phosphorus ligands and/or free phosphorus ligands.

7. The process according to claims 1 to 6, wherein the mononitrile is 3-pentenenitrile and the dinitrile is adiponitrile.

8. The process according to claims 1 to 7, wherein the reaction effluent is obtained by reacting 3-pentenenitrile with hydrogen cyanide in the presence of at least one nickel(0) complex having phosphorus ligands, if appropriate in the presence of at least one Lewis acid.

9. The process according to claims 1 to 8, wherein the hydrocarbon H used is cyclohexane, methylcyclohexane, n-heptane or n-octane.

10. The process according to claims 1 to 9, wherein the hydrocarbon H is mixed with the concentrated reaction effluent in step b) in a stirred vessel or a pumped circulation system.

11. The process according to claims 1 to 10, wherein the hydrocarbon H is part of stream II.

12. The process according to claims 1 to 11, wherein the extraction in step c) is carried out in a countercurrent extraction column which is equipped with sheet metal packings as dispersing elements.

13. The process according to claims 1 to 12, wherein the extraction in step c) is carried out in countercurrent in a compartmented, stirred extraction column.

14. The process according to claims 1 to 13, wherein stream I is conducted through a delay zone after step b) and before step c).

15. The process according to claims 1 to 14, wherein the average residence time of stream I in the delay zone is at least 1 min.

16. The process according to claims 1 to 15, wherein the flow rate of stream I in all pipelines used in the process is at least 2 m/s.

17. The process according to claims 1 to 16, wherein at least one of the three streams, concentrated reaction effluent, hydrocarbon H and stream I, is adjusted to a temperature T* which is at least 5°C below the temperature T.

## Revendications

1. Procédé de séparation extractive de catalyseurs dissous de manière homogène à partir d'un effluent réactionnel d'une hydrocyanation de mononitriles insaturés pour donner des dinitriles, par extraction grâce à un hydrocarbure K, **caractérisé par** les étapes consistant à :
a) concentrer l'effluent réactionnel avant l'étape b) grâce à une distillation à des pressions de 0,1 à 5000 mbars et à une température de 10 à 150°C,
b) ajouter un hydrocarbure K à un effluent réactionnel concentré, un courant I étant obtenu, et
c) alimenter le courant I sans séparation préalable des phases liquides dans un dispositif d'extraction et extraire à une température T avec l'hydrocarbure K, un courant II, contenant l'hydrocarbure K enrichi avec le catalyseur, et un courant III pauvre en catalyseur étant obtenus.

2. Procédé selon la revendication 1, **caractérisé en ce que** l'effluent réactionnel concentré avant l'étape b), ou le courant I pendant ou après l'étape b) ou pendant l'étape c), est traité avec de l'ammoniac ou une amine aromatique ou aliphatique primaire, secondaire ou tertiaire.

3. Procédé selon les revendications 1 à 2, **caractérisé en ce que** l'ammoniac ou l'amine est ajouté en commun avec l'hydrocarbure K.

4. Procédé selon l'une quelconque des revendications 1 à 3, **caractérisé en ce que** les matières solides précipitant dans l'étape b) sont séparées avant l'étape c) à partir du courant I.

5. Procédé selon l'une quelconque des revendications 1 à 4, **caractérisé en ce que** la séparation de matières solides est mise en oeuvre avant, pendant ou après le traitement de l'effluent réactionnel ou du courant I.

6. Procédé selon l'une quelconque des revendications 1 à 5, **caractérisé en ce que** le catalyseur est représenté par des complexes de nickel (0) comprenant des ligands contenant du phosphore, et/ou des ligands libres contenant du phosphore.

7. Procédé selon l'une quelconque des revendications 1 à 6, **caractérisé en ce que** le mononitrile est du 3-pentènenitrile et le dinitrile est de l'adipodinitrile.

8. Procédé selon l'une quelconque des revendications 1 à 7, **caractérisé en ce que** l'effluent réactionnel est obtenu par réaction de 3-pentènenitrile avec de l'acide hydrocyanique en présence d'au moins un complexe de nickel (0) comprenant des ligands contenant du phosphore, éventuellement en présence d'au moins un acide de Lewis.

9. Procédé selon l'une quelconque des revendications 1 à 8, **caractérisé en ce que** du cyclohexane, du méthylcyclohexane, du n-heptane ou du n-octane est utilisé en tant qu'hydrocarbure K.

10. Procédé selon l'une quelconque des revendications 1 à 9, **caractérisé en ce que** l'hydrocarbure K est mélangé dans l'étape b) avec l'effluent réactionnel concentré dans une mélangeuse d'agitation ou un circuit de transvasage.

11. Procédé selon l'une quelconque des revendications 1 à 10, **caractérisé en ce que** l'hydrocarbure K est un courant partiel du courant II.

12. Procédé selon l'une quelconque des revendications 1 à 11, **caractérisé en ce que** l'extraction est mise en oeuvre dans l'étape c) dans une colonne d'extraction à contre-courant, qui est équipée de garnissages de tôle en tant qu'éléments dispersants.

13. Procédé selon l'une quelconque des revendications 1 à 12, **caractérisé en ce que** l'extraction est mise en oeuvre dans l'étape c) à contre-courant dans une colonne d'extraction agitée et compartimentée.

14. Procédé selon l'une quelconque des revendications 1 à 13, **caractérisé en ce que** le courant I est guidé après l'étape b) et avant l'étape c) grâce à un parcours de temps de séjour.

15. Procédé selon l'une quelconque des revendications 1 à 14, **caractérisé en ce que** le temps de séjour moyen du courant I dans le parcours de temps de séjour est d'au moins 1 min.

16. Procédé selon l'une quelconque des revendications 1 à 15, **caractérisé en ce que** la vitesse d'écoulement du courant I dans toutes les conduites utilisées dans le procédé est d'au moins 2 m/s.

17. Procédé selon l'une quelconque des revendications 1 à 16, **caractérisé en ce qu'**au moins un des trois courants parmi l'effluent réactionnel concentré, l'hydrocarbure K et le courant I est mis en température à une température T* qui se situe au moins 5 °C en dessous de la température T.
